# EUROPÄISCHE PATENTSCHRIFT

(11) **EP 3 427 681 B1**
(45) Veröffentlichungstag und Bekanntmachung des Hinweises auf die Patenterteilung: **02.03.2022**
(21) Anmeldenummer: 18175958.0
(22) Anmeldetag: 05.06.2018
(51) Int. Cl.: A61B 18/14, A61B 17/32, A61B 18/00, A61B 17/00, A61B 18/20

(54) **MEDIZINISCHES INSTRUMENT ZUM ABTRAGEN VON GEWEBE**
MEDICAL INSTRUMENT FOR REMOVING TISSUE
INSTRUMENT MÉDICAL DESTINÉ À L'ENLÈVEMENT D'UN TISSU

(30) Priorität: 13.07.2017 DE 102017115778
(43) Veröffentlichungstag der Anmeldung: 16.01.2019
(73) Patentinhaber: Karl Storz SE & Co. KG, 78532 Tuttlingen (DE)
(72) Erfinder: Rehbein, Stefan, 78532 Tuttlingen (DE); Wittke, Uwe, 78532 Tuttlingen (DE); Löffler, Oliver, 78532 Tuttlingen (DE)

(56) Entgegenhaltungen:
- WO-A1-2016/018457
- DE-A1- 4 301 249
- US-A- 6 024 751
- US-A1- 2012 150 179

## Beschreibung

Die Erfindung betrifft allgemein ein medizinisches Instrument für die minimal-invasive Chirurgie, insbesondere ein medizinisches Instrument für die Resektoskopie, zur kalten Enukleation (Ausschälung) von Gewebe, insbesondere von Prostatagewebe, durch einen minimal-invasiven chirurgischen Eingriff.

### Hintergrund der Erfindung

Aus der modernen Medizin sind minimal-invasive Eingriffe heutzutage nicht mehr wegzudenken. Im Bereich der Resektoskopie, bei der schädliches oder beschädigtes Gewebe (z.B. aus der Prostata) entfernt wird, kommen dabei insbesondere Resektoskope zur Anwendung, die über ein Hochfrequenz-Schneidemittel verfügen, mit dem durch Hochfrequenzströme Gewebe abgetragen wird. Ein Vorteil beim Abtragen von Gewebe mittels Hochfrequenzströmen ist, dass Blutungen am Operationsort rasch gestillt werden können.

DE 2006 039 696 A1 offenbart eine Vorrichtung zur Resektion und / oder Ablation von organischem Gewebe mittels Hochfrequenzstrom. Dabei umfasst die Vorrichtung eine mit einer Hochfrequenzspannung beaufschlagbare Schlinge und einen Schlingenträger, der als zylinderförmiges Hohlrohr ausgebildet ist. Die Schlinge ist an einem distalen Ende des Schlingenträgers angeordnet, wobei ein Schlingenendabschnitt der Schlinge und ein distaler Verbindungselementabschnitt eine keilförmige Schneide bilden, die mit der Hochfrequenzspannung beaufschlagbar ist. Weil die Schlinge aus einem elektrisch leitenden Draht ausgebildet ist, kann diese sich schon bei geringer Krafteinwirkung am Operationsort verformen, was ein präzises Abtragen von Gewebe erschwert.

DE 693 33 489 T2 offenbart eine medizinische Sondenvorrichtung, die einen Katheter und ein biegsames Stilett umfasst, wobei das Stilett in einem entsprechenden Führungsgehäuse geführt wird. Zum Abtragen von Gewebe kann eine Hochfrequenz-Elektrode (teilweise auch Nadel genannt) an dem Stilett angeordnet sein. Obwohl die genannte Vorrichtung eine Vielzahl an zusätzlichen Möglichkeiten mit sich bringt, ist jedoch auch hier ein mechanisches Abtragen von Gewebe nicht möglich, da die Hochfrequenz-Elektrode sich schon bei geringer Krafteinwirkung am Operationsort verformt.

EP 2 298 204 B1 der Anmelderin offenbart ein medizinisches Instrument für die bipolare Elektrochirurgie, mit einem Außenschaft, an dessen distalem Ende eine elektrisch leitende Schneide angeordnet ist, die gegenüber dem Außenschaft isoliert ist, und einem Arbeitseinsatz, der in dem Außenschaft aufgenommen und in diesem axial hin und her bewegt werden kann und der an seinem distalen Ende einen Haken mit einer proximal nach außen gerichteten Schneidkante aufweist. In einer ausgefahrenen Stellung des Arbeitseinsatzes kann von dem Haken Gewebe ergriffen und durch Einziehen des Arbeitseinsatzes in den Außenschaft in Richtung auf die Schneide zu bewegt werden. Bei Beaufschlagen von Haken und Schneide mit einem Hochfrequenzstrom wird dazwischen gehaltenes Gewebe koaguliert. Durch weiteres Einziehen des Arbeitseinsatzes in den Außenschaft kann Gewebe auch durchtrennt werden. Weil die Schneidkanten von Haken und Schneide unter einem Winkel zueinander stehen, kann zwischen den Schneidkanten von Haken und Schneide eine scherenartige Schnittbewegung ausgeführt werden. Aufbau und Bedienverhalten dieses medizinischen Instruments sind jedoch vergleichsweise kompliziert.

Außerdem sind aus dem Stand der Technik auch Schneideinstrumente für die minimal-invasive Chirurgie bekannt, die an einem Außenschaft eines Endoskops, Resektoskops oder einer Nadel befestigt sind.

US 6 245 011 B1 offenbart ein solches endoskopisches Schneideinstrument, das eine radial verstellbare Schneide umfasst, die an einem distalen Ende des Außenschafts des Instruments angeordnet ist und radial über den Außenschaft hinaus ausgefahren werden kann, sodass mit diesem durch eine Drehbewegung in einer umlaufenden Richtung geschnitten werden kann.

WO 2016/018457 A1 offenbart ein endoskopisches Schneidesystem mit zwei Schneideelementen: gegeneinander drehbare Röhren und ein spitzes Ende zum Schneiden von Gewebe.

Zusammenfassend haben die Instrumente nach dem Stand der Technik insbesondere den Nachteil, dass kein mechanisches Abtragen von Gewebe in einer axialen Richtung möglich ist.

### Beschreibung der Erfindung

Es ist daher eine Aufgabe der vorliegenden Erfindung, ein medizinisches Instrument zum mechanischen Abtragen bzw. Abschaben von Gewebe durch einen minimal-invasiven chirurgischen Eingriff bereitzustellen, mit dem in einer axialen Bewegung gezielt Gewebe abgeschabt bzw. abgetragen werden kann, wobei dieses Instrument sicher an den Operationsort geführt werden kann.

Die Aufgabe wird durch das erfindungsgemäße medizinische Instrument nach Anspruch 1 gelöst. Weitere vorteilhafte Ausführungsformen ergeben sich durch die Unteransprüche.

Die Offenbarung betrifft ein medizinisches Instrument zum mechanischen Abtragen bzw. Abschaben von Gewebe durch einen minimal-invasiven chirurgischen Eingriff, das einen hohlen Außenschaft und einen Innenschaft umfasst, wobei an einem proximalen Ende des medizinischen Instruments ein Bedienabschnitt angeordnet ist, der ausgelegt ist, um den hohlen Außenschaft geeignet am Operationsort zu platzieren. Erfindungsgemäß ist unmittelbar an einem distalen Ende des Innenschafts ein Aufsatz zum mechanischen Abtragen von Gewebe angeordnet, wobei der Aufsatz radial nicht über ein von den Innenseiten bzw. der Innenumfangswand des Außenschafts ausgebildetes Innenprofil hinausragt und wobei zum Abtragen von Gewebe das distale Ende des Aufsatzes axial über das distale Ende des hohlen Außenschafts hinausragt.

Hierzu kann der Innenschaft grundsätzlich relativ zu dem hohlen Außenschaft unbeweglich sein, um gemeinsam mit diesem zum Platzieren des Aufsatzes zum Abtragen von Gewebe am Operationsort platziert zu werden. Gemäß einer bevorzugten Ausführungsform ist jedoch der Innenschaft relativ zu dem hohlen Außenschaft axial verstellbar

Im Folgenden werden die in der medizinischen Terminologie üblichen Begriffe "proximal" und "distal" verwendet. In Bezug auf das medizinische Instrument bedeutet dabei "proximal" auch zum Operierenden hin, d.h. vom Operationsort weg, und "distal" zum Operationsort hin, d.h. vom Operierenden weg. Mit anderen Worten also vorne und hinten in Längsrichtung des medizinischen Instruments vom Bedienabschnitt aus betrachtet.

Wie zuvor beschrieben ist der Außenschaft hohl, d.h. rohrförmig ausgebildet, insbesondere zylindrisch, ggf. oval oder elliptisch, um den Innenschaft darin zu führen. Der Außenschaft wird zum Einführen bzw. Platzieren in den menschlichen Körper verwendet. Hierzu kann der proximale Bedienabschnitt bevorzugt einen Griff umfassen, der insbesondere auch ein Platziergriff sein kann und so mit dem Außenschaft gekoppelt ist, dass ein distales Ende des medizinischen Instruments präzise an den Operationsort im menschlichen Körper geführt werden kann. Der Außenschaft ist dabei als Bestandteil eines mikroinvasiven Instruments gedacht, wobei als solche Instrumente mikroinvasive Instrumente, Endoskope, Nadeln und bevorzugter Weise auch Resektoskope in Frage kommen. Dabei kann zusammen mit diesem Instrument bzw. in einem lichten Querschnitt dieses Instruments ein Endoskop mit eingeführt werden.

Der Innenschaft des medizinischen Instruments ist gemäß einer weiteren Ausführungsform im Außenschaft längsverschieblich geführt, kann dabei aber in einer oder mehreren Positionen, ggf. auch stufenlos, axial arretiert werden. Dabei ist eine enge Führung denkbar, bei der die Außenwand des Innenschafts eng an der Innenwand des Außenschafts anliegt und insbesondere an dieser gleitet. Alternativ sind auch andere Arten der Führung denkbar, bei denen der Innenschaft nicht eng anliegend in dem Außenschaft geführt wird, sondern zusätzliche Führungsmittel zum präzisen Führen des Innenschaftes in Längsrichtung in dem hohlen Außenschaft vorgesehen sind. Der Innenschaft kann dabei sowohl einen runden, beispielsweise kreisrunden Querschnitt als auch einen elliptischen Querschnitt aufweisen.

Ein wichtiger Bestandteil des medizinischen Instruments ist der an dem distalen Ende befestigte Aufsatz zum mechanischen Abtragen bzw. Abschaben, Abheben oder Ablösen, im Folgenden als Abtragen bezeichnet, von Gewebe, wobei der Aufsatz dabei bevorzugt in Form einer in axialer und radialer Richtung vorstehenden Nase ausgebildet ist. Durch diesen Aufsatz wird ein mechanisches Abtragen von Gewebe durch Bewegen des Aufsatzes relativ zu dem abzutragenden Gewebe am Operationsort ermöglicht. Hierzu wird der Innenschaft, und damit auch der Aufsatz, bevorzugt nach vorne und zurückbewegt wird. Durch die solide, mechanisch stabile Konstruktion des Aufsatzes kann hierbei eine relativ große Kraft auf das distale Ende des Aufsatzes aufgebracht werden. Dadurch können auch feste Gewebeteile (relativ) schnell abgetragen werden. Somit eignet sich der Aufsatz zum weitflächigen Abtragen von ungewünschtem oder auch totem oder (schlecht durchblutetem) geschädigtem Gewebe.

Zum Abtragen von Gewebe mittels des Aufsatzes kann dabei grundsätzlich eine Verstellung des Außenschaftes gemeinsam mit dem Innenschaft erfolgen. In einem Spezialfall erfolgt jedoch die Bewegung des Aufsatzes durch Verstellen des Innenschaftes relativ zu dem hohlen Außenschaft durch Betätigen des Bedienabschnitts. Eine Inzissionsöffung des Außenschaftes dient dabei als quasi ortsfester Bezugsbereich, der geeignet in Bezug zu dem Operationsort platziert wird, insbesondere auch unter einem geeigneten Winkel des Aufsatzes relativ zu dem abzutragenden Gewebe, und das eigentliche Abtragen des Gewebes erfolgt durch Verstellung des Innenschaftes relativ zu dem holen Außenschaft mittels des Bedienabschnitts, der hierzu auch über eine geeignete Über- oder Untersetzung verfügen kann, um eine Bewegung eines Unterabschnitts des Bedienabschnitts relativ zu einem relativ zu diesem verstellbaren weiteren Unterabschnitts des Bedienabschnitts zu über- oder untersetzen, was insgesamt eine sehr präzise Verstellung des Aufsatzes ermöglicht.

Falls der Innenschaft einen kreisrunden Querschnitt aufweist, ist zusätzlich eine Drehbewegung des Innenschaftes relativ zu dem Außenschaft möglich. Diese Drehbewegung kann z.B. zum seitlichen Schaben, Abtragen oder Schneiden von Gewebe verwendet werden oder lediglich einer weiteren Positionierung des Aufsatzes dienen.

In einer bevorzugten Ausführungsform ist der Bedienabschnitt so mit dem Innenschaft gekoppelt, dass der Aufsatz vollständig in den hohlen Außenschaft zurückgefahren werden kann und zum Abtragen von Gewebe der Aufsatz zumindest abschnittsweise axial über ein distales Ende des hohlen Außenschafts hinausragt.

Durch dieses Zurückfahren des Innenschaftes und damit auch des Aufsatzes zum Abtragen von Gewebe kann gewährleistet werden, dass der Aufsatz zum Abtragen von Gewebe sicher an den Operationsort geführt werden kann, ohne auf dem Weg dorthin gesundes Gewebe zu beschädigen. Am Operationsort angelangt kann dann der Aufsatz zum Abtragen von Gewebe axial ausgefahren werden, um mit dem Abtragen zu beginnen. Zum Abtragen von Gewebe muss das distale Ende des Aufsatzes zumindest teilweise aus dem Außenschaft herausgefahren sein, um mit dem Gewebe in Kontakt zu gelangen, das abzutragen ist. In bevorzugter Weise ragt jedoch der gesamte Aufsatz in seiner maximal ausgefahrenen Stellung aus dem hohlen Außenschaft heraus.

In einer bevorzugten Ausführungsform umfasst der Aufsatz des medizinischen Instruments einen sich geradlinig erstreckenden Abschnitt, der mit dem distalen Ende des Innenschaftes verbunden ist. Dadurch kann der Aufsatz bei einer vorgegebenen axialen Verstellung des Innenschaftes relativ zu dem Außenschaft maximal aus dem hohlen Außenschaft herausragen, um Gewebe abzutragen. Bevorzugt erstreckt sich ein distales Ende des Aufsatzes geneigt zu dem geradlinigen Abschnitt. Mit anderen Worten ist also das distale Ende des Aufsatzes zu einer Mittellinie des medizinischen Instruments hin geneigt. Durch diese Formgestaltung sticht der Aufsatz beim Verstellen des Innenschaftes nicht einfach in das Gewebe vor dem distalen Ende des hohlen Außenschafts hinein. Vielmehr kann der geneigte distale Abschnitt kontrolliert über einen Gewebeabschnitt gleitend bewegt werden, um Gewebe abzutragen, insbesondere abzuschälen, wobei der Ort des Aufsatzes jederzeit durch eine geeignete Abbildungsoptik kontrolliert werden kann.

Gemäß einer bevorzugten weiteren Ausführungsform erstreckt sich das sich geneigt erstreckende distale Ende des Aufsatzes nicht über die vorgenannte Instrumentenmittellinie bzw. Mittellinie des hohlen Außenschafts hinaus, sodass nur maximal eine Hälfte des Gewebebereichs in Verlängerung des hohlen Außenschafts mit dem Aufsatz erreicht werden kann. Die andere Hälfte dieses Gewebebereichs lässt sich natürlich durch einfaches Verdrehen des hohlen Außenschafts erreichen. Insgesamt kann so ein noch präziseres Abtragen von Gewebe erreicht werden.

In einer bevorzugten Ausführungsform erstreckt sich der Aufsatz somit bis maximal 50% und in besonders bevorzugter Weise bis 40% des Innendurchmessers des hohlen Außenschaftes hin zur Instrumentenmittellinie.

Nach einer weiteren Ausführungsform schließt sich dem geradlinigen Abschnitt des Aufsatzes ein bogenförmig gekrümmter oder abgewinkelter Abschnitt an, an dessen distalem Ende eine Abtragekante vorgesehen ist, die sich bevorzugt rechtwinklig zur Unter- oder Oberseite des distalen Endes des bogenförmig gekrümmten oder abgewinkelten Abschnitts erstreckt. Die nasenförmige Konstruktion des Aufsatzes ist ähnlich zu einer (Schlitten-) Kufe ausgebildet und nach vorne hin abgewölbt bzw. abgewinkelt. Genauer gesagt ist also das distale Ende unter einem Winkel zur Mittellinie des Außenschaftes hin abgewinkelt, wobei der gekrümmte oder abgewinkelte Abschnitt mit dem geradlinigen Abschnitt einen Winkel einschließt. Dieser Winkel kann zwischen 115° und 155° liegen, bevorzugter zwischen 120° und 150° liegen und noch bevorzugter zwischen 130° und 140° liegen. Die vorgenannte Abtragekante erstreckt sich somit ebenfalls geneigt zur Mittellinie des hohlen Außenschafts, bevorzugt unter einem Winkel von etwa 45° zur Mittellinie des hohlen Außenschafts, was das Abtragen von Gewebe effizienter macht und insbesondere ein effektives Ausschälen ermöglicht. Hierzu ist die Abtragekante möglichst rechtwinklig ausgebildet, mit einem möglichst geringen Krümmungsradius der Abtragekante.

Durch diese vorteilhafte Konstruktion ist der Operierende in der Lage, mit dem geradlinigen proximalen Teil des Aufsatzes über Gewebe zu gleiten, und überstehendes Gewebe mit der abgewinkelten Abtragekante der Vorrichtung abzutragen, falls dieses Gewebe übersteht. Das heißt, dass ein Einschneiden in gesundes Gewebe, das in einer tieferen Gewebeschicht liegt, erschwert wird, wodurch unerwünschte Verletzungen effizient vermieden werden können. Folglich ermöglicht die Vorrichtung ein gezieltes Abtragen insbesondere von in Bezug zur Mittellinie des Außenschaftes überstehendem Gewebe, insbesondere von Gewebe, das sich zumindest abschnittsweise senkrecht zu dieser Mittellinie erstreckt. Die Winkelwahl kann dabei entsprechend der abzutragenden Gewebeschicht geeignet gewählt werden.

Nach einer weiteren Ausführungsform erstreckt sich die Abtragekante des medizinischen Instruments im Wesentlichen senkrecht zu einer Unterseite des distalen Endes. Diese Ausführungsform ist insbesondere zum Abtragen von leicht abtrennbarem Gewebe vorteilhaft, da obere Gewebeschichten sicher abgeschabt werden können. Nach weiteren Ausführungsformen sind aber auch klingen- bzw. schneidenähnliche Abtragekanten möglich, wobei diese insbesondere dann zum Einsatz kommen, wenn festeres Gewebe abgetrennt werden soll, z.B., ggf. karzinomes, Gewebe in der Prostata.

Nach einer bevorzugten Ausführungsform liegt die axiale Länge des Aufsatzes, gerechnet vom distalen Ende des Innenschaftes, im Bereich zwischen 1 mm und 16 mm, bevorzugter zwischen 3 mm und 13 mm und noch bevorzugter zwischen 5 mm und 11 mm, wobei auch andere Längen denkbar sind. Bei der Wahl der axialen Länge sind zwei Faktoren zu berücksichtigen. Ein längerer Aufsatz ermöglicht es, einen größeren Bereich abzuschaben und, bei positioniertem Außenschaft, weiter in das Gewebe vorzudringen. Jedoch resultiert ein zu langer Außenschaft auch in zunehmender Instabilität und sinkender Bedienpräzision des Aufsatzes. Dies liegt daran, dass sich der Operateur normalerweise mit einer Abbildungsoptik orientiert, die (in diesem Fall) im Innenschaft angeordnet ist; falls sich die Abtragekante zu weit von der Kamera entfernt, kann daher der Abtragebereich nicht mehr ausreichend eingesehen werden oder außerhalb des Tiefenschärfebereichs der Abbildungsoptik liegen. Der vorgenannte Bereich ist ein resultierender optimierter Bereich.

Nach einer weiteren Ausführungsform ist der Aufsatz an das distale Ende des Innenschafts angeschweißt, insbesondere im Bereich einer Aussparung, die am distalen Ende des Innenschaftes ausgebildet ist. Dies ermöglicht eine exzellente Stabilität des Aufsatzes an dem medizinischen Instrument, um ein Verlieren oder seitliches Ausweichen des Aufsatzes während einer Operation zuverlässig auszuschließen. Andere nicht-lösbare oder sogar lösbare Befestigungen sind jedoch grundsätzlich möglich, insbesondere ein lösbarer Verrastungsmechanismus, etwa um einen einfachen Austausch des Aufsatzes durch einen anderen Aufsatz mit einer anderen Formgestaltung zu ermöglichen.

Nach einer weiteren Ausführungsform ist der Innenschaft längsverschieblich in dem hohlen Außenschaft geführt, wobei der Bedienabschnitt weiterhin ausgelegt ist, um die Position des Innenschaftes relativ zu dem hohlen Außenschaft zu verstellen. Dabei kann der Bedienabschnitt insbesondere so mit dem Innenschaft gekoppelt sein, dass der Aufsatz vollständig in den hohlen Außenschaft zurückgefahren werden kann, etwa zum Einführen des Aufsatzes in menschliches Gewebe, und dass der Aufsatz zum Abtragen von Gewebe zumindest abschnittsweise axial über das distale Ende des hohlen Außenschafts hinausragt.

Dadurch kann eine Beschädigung von Gewebe beim Einführen des Aufsatzes in menschliches Gewebe effektiv vermieden werden.

Gemäß einer weiteren Ausführungsform kann hierzu der Innenschaft in dem Außenschaft längsverschieblich geradgeführt sein, wodurch ein unkontrolliertes Verdrehen des Innenschaftes relativ zu dem Außenschaft effizient verhindert ist und der Aufsatz noch präziser zum Operationsort geführt und dort geeignet platziert werden kann. In vorteilhaft einfacher Weise lässt sich eine solche Geradeführung dadurch erzielen, dass das Außenprofil des Innenschaftes nicht rotationssymmetrisch ausgebildet ist, insbesondere in Gestalt eines ovalen oder elliptischen Außenprofils, wobei das Innenprofil des Außenschaftes korrespondierend zum Außenprofil des Innenschaftes ausgebildet ist.

Dabei ist der Innenschaft des medizinischen Instruments als hohles Rohr ausgebildet. Dadurch ist es möglich, weitere Instrumente, z.B. HF-Elektroden, Kameras, Abbildungsoptiken oder weitere Schneideinstrumente, an den Operationsort zu führen. Somit werden also Kombiinstrumente ermöglicht.

Dabei ist weiterhin ein längliches Aufhahmeelement mit einer Aussparung oder Aufnahme zur vorübergehenden Aufnahme des Aufsatzes zum mechanischen Abtragen von Gewebe vorgesehen, wobei das längliche Aufnahmeelement relativ zu dem hohlen Innenschaft axial zwischen einer ausgefahrenen Stellung, in der der Aufsatz zum mechanischen Abtragen von Gewebe in der Aussparung oder Aufnahme vorübergehend aufgenommen ist, und einer eingefahrenen Stellung axial verstellbar ist, in der der Aufsatz zum mechanischen Abtragen von Gewebe über das distale Ende des hohlen Außenschafts axial hinausragt und zum Abtragen von Gewebe zugänglich ist.

Nach einer weiteren bevorzugten Ausführungsform ist des Weiteren am distalen Ende des Innenschaftes ein HF-Koagulationsaufsatz zum Koagulieren von Gewebe durch Anlegen einer Hochfrequenzspannung (HF) vorgesehen. Dieser HF-Koagulationsaufsatz kann fest mit dem Instrument verbunden sein, kann jedoch grundsätzlich auch relativ zu dem hohlen Innenschaft axial verstellbar sein oder nachträglich, etwa nach Einführen des Instrumentenaufsatzes in das menschliche Gewebe und nach Platzieren desselben am Operationsort, in den hohlen Innenschaft eingeführt werden. Das medizinische Instrument ist also zu einem Kombiinstrument erweitert, das verschiedene Operationsmodi ermöglicht. Dies kann insbesondere von Nutzen sein, falls durch das mechanische Abtragen Gewebe abgetragen wird und Blutungen entstehen. Durch das Koagulieren mit dem HF-Koagulationsaufsatz können die besagten Blutungen dann einfach gestoppt werden, ohne dass hierzu zunächst der Innenschaft mit dem Aufsatz aus dem hohlen Außenschafts herausgeführt werden und durch einen Koagulationsaufsatz ersetzt werden muss.

Nach einer weiteren Ausführungsform weist der HF-Koagulationsaufsatz hierzu eine mit einer Hochfrequenzspannung beaufschlagbare Schlinge zum Koagulieren von Gewebe auf, die axial weniger weit von dem distalen Ende des Innenschaftes vorsteht als der Aufsatz zum mechanischen Abtragen von Gewebe und die zu dem Innenschaft elektrisch isoliert ist. Dadurch ist in einfacher Weise ein mechanischer Schutz der Schlinge durch den Aufsatz realisiert, etwa beim Einführen des Instrumentenaufsatzes in menschliches Gewebe. Dabei kann die Schlinge einen Verbindungsabschnitt und eine bogenförmig gekrümmte Schleife am distalen Ende des Verbindungsabschnitts aufweisen, wobei sich die bogenförmig gekrümmte Schleife im Wesentlichen parallel zum distalen Ende des Innenschaftes erstreckt, insbesondere geneigt zur Instrumentenmittellinie, wodurch eine axiale Verstellungslänge zum Herausfahren des HF-Koagulationsaufsatzes über das distale Ende des hohlen Innenschafts hinaus in einfacher Weise minimiert werden kann.

Nach einer weiteren Ausführungsform erstrecken sich die bogenförmige Schleife und das distale Ende des Innenschaftes, in einer Seitenansicht des distalen Endes des medizinischen Instruments betrachtet, geneigt und hin zum proximalen Ende des hohlen Innenschafts, wodurch in einfacher Weise ein keilförmig geneigtes Arbeitsende des HF-Koagulationsaufsatzes zum Koagulieren von Gewebe vor dem distalen Ende des Innenschaftes realisiert werden kann.

Nach einer weiteren Ausführungsform ist alternativ zu dem vorgenannten HF-Koagulationsaufsatz am distalen Ende des Innenschaftes ein Laser-Koagulationsaufsatz zum Koagulieren von Gewebe durch Laserstrahlung vorgesehen. Der Laser-Koagulationsaufsatz kann dabei axial von dem distalen Ende des Innenschafts vorstehen, wobei auch nach dieser Ausführungsform ein distales Ende des Laser-Koagulationsaufsatzes weniger weit von dem distalen Ende des Innenschaftes vorsteht als der Aufsatz zum mechanischen Abtragen von Gewebe. Dieser Laser-Koagulationsaufsatz kann fest mit dem Instrument verbunden sein, kann jedoch grundsätzlich auch relativ zu dem hohlen Innenschaft axial verstellbar sein oder nachträglich, etwa nach Einführen des Instrumentenaufsatzes in das menschliche Gewebe und nach Platzieren desselben am Operationsort, in den hohlen Innenschaft eingeführt werden.

### Kurze Beschreibung der Zeichnungen

Im Folgenden werden bevorzugte Ausführungsformen unter Bezugnahme auf die beigefügten Zeichnungen beschrieben. Es zeigen:
- Fig. 1: eine schematische Ansicht eines medizinischen Instruments nach einer bevorzugen Ausführungsform der vorliegenden Erfindung;
- Fig. 2a: eine schematische Ansicht des distalen Bereiches des medizinischen Instruments mit einer vorne gekrümmten Abtragekante nach einer bevorzugen Ausführungsform der vorliegenden Erfindung;
- Fig. 2b: eine schematische Ansicht des distalen Bereiches des medizinischen Instruments mit einer vorne gekrümmten Abtragekante gemäß der Fig. 1;
- Fig. 3a: zeigt eine schematische Ansicht eines medizinischen Instruments mit einem zusätzlichen HF-Koagulationsaufsatz nach einer weiteren Ausführungsform der vorliegenden Erfindung;
- Fig. 3b: in einer stark vergrößerten Perspektivansicht den distalen Bereich des medizinischen Instruments gemäß der Fig. 3a;
- Fig. 3c: in einer stark vergrößerten Seitenansicht den distalen Bereich des medizinischen Instruments gemäß der Fig. 3a;
- Fig. 4a: eine schematische Ansicht eines medizinischen Instruments mit einem zusätzlichen Laser-Koagulationsaufsatz nach einer weiteren Ausführungsform der vorliegenden Erfindung;
- Fig. 4b: eine stark vergrößerte Perspektivansicht des distalen Bereiches des medizinischen Instruments gemäß der Fig. 4a;
- Fig. 4c: eine stark vergrößerte Seitenansicht des distalen Bereiches des medizinischen Instruments gemäß der Fig. 4a;
- Fig. 5a: in einer Perspektivansicht ein medizinisches Instrument gemäß einer weiteren Ausführungsform der vorliegenden Erfindung;
- Fig. 5b: den Ausschnitt nach der Fig. 5a in einer stark vergrößerten Perspektivansicht;
- Fig. 6a: in einer stark vergrößerten Perspektivansicht den distalen Bereich eines medizinischen Instruments gemäß einer weiteren Ausführungsform der vorliegenden Erfindung;
- Fig. 6b: den distalen Bereich des medizinischen Instruments gemäß der Fig. 6a in einer Vorderansicht;
- Fig. 7a: in einer stark vergrößerten Perspektivansicht den distalen Bereich eines medizinischen Instruments gemäß einer weiteren Ausführungsform der vorliegenden Erfindung; und
- Fig. 7b: den distalen Bereich des medizinischen Instruments gemäß der Fig. 7a in einer Vorderansicht.

In den Figuren bezeichnen identische Bezugszeichen identische oder im Wesentlichen gleichwirkende Elemente oder Elementgruppen.

### Ausführliche Beschreibung der Zeichnungen

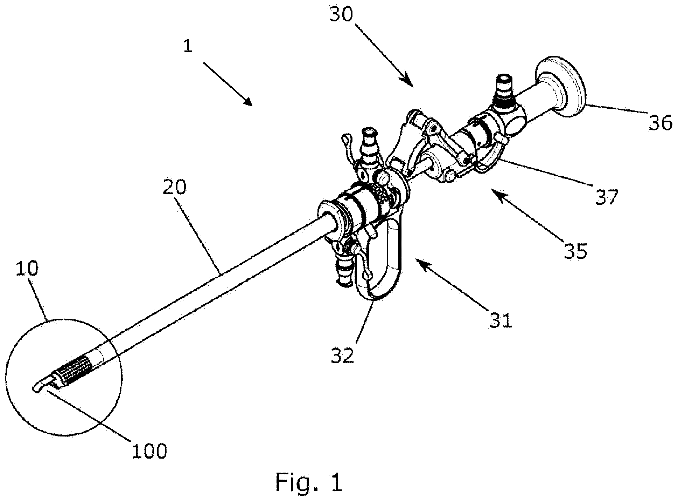

Fig. 1 zeigt ein medizinisches Instrument 1 zum Abtragen von Gewebe, insbesondere von Prostatagewebe, durch einen minimal-invasiven chirurgischen Eingriff nach einer bevorzugten Ausführungsform der vorliegenden Erfindung. Dabei weist das medizinische Instrument 1 im Wesentlichen drei Abschnitte auf: einen vorderen oder auch distalen Abschnitt 10, einen sich an einem hinteren oder proximalen Ende befindlichen Bedienabschnitt 30, und einen durch den Schaft 20 ausgebildeten Abschnitt, der sich zwischen den zuvor genannten Abschnitten erstreckt und jeweils mit diesen verbunden ist.

Das distale Ende 10 befindet sich am vorderen Ende des Schafts 20 und umfasst insbesondere eine vordere Öffnung des Schafts 20. An einer Außenwand des Schafts 20 ist ein nasenförmiger Aufsatz 100 angeordnet, der zum Abtragen von Gewebe dient.

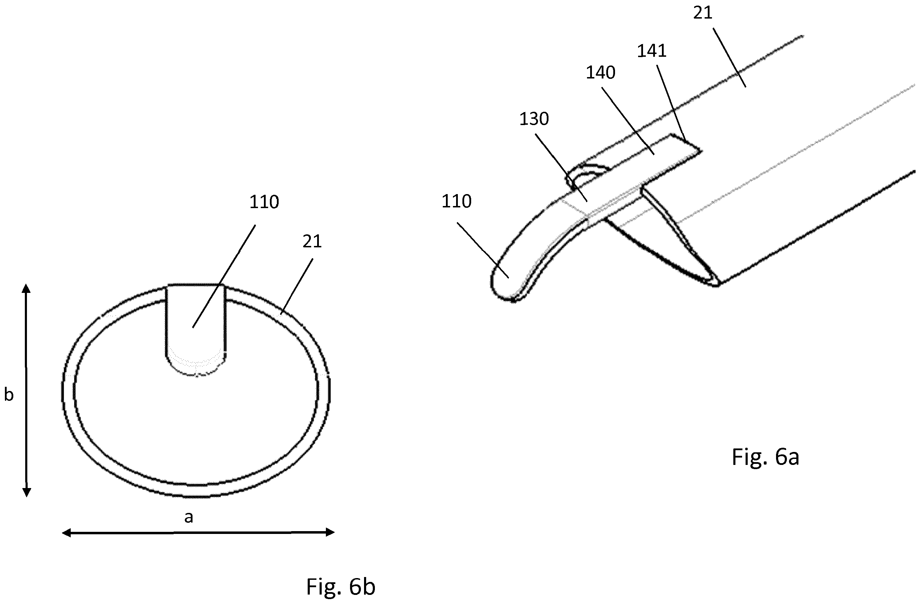

Der Schaft 20 weist eine im Wesentlichen zylindrische Form auf und hat eine angemessene Länge und Dimension, um in einen menschlichen Körper eingeführt zu werden. In einer alternativen Gestaltung kann der Schaft auch eine ovale oder andere Rohrform aufweisen, wie nachfolgend anhand der Figuren 6a bis 7b beschrieben. Mit dem Schaft 20 können in einem minimal-invasiven chirurgischen Eingriff Operationsinstrumente an den Ort einer Operation in den menschlichen Körper eingeführt werden.

Der Bedienabschnitt 30 weist im Wesentlichen zwei Teilabschnitte auf, nämlich einen Platziergriffabschnitt 31 und einen Operationsgriffabschnitt 35, die längsbeweglich miteinander über eine Übertragungswelle, zum Übertragen einer Kraft, und ein Positionierungs-Scharnier, zum Positionieren und zum Führen des Schafts, verbunden sind.

Am sich am distalen Ende des Bedienabschnitts 30 befindlichen Platziergriffabschnitt 31 ist insbesondere ein Platziergriff 32 angeordnet, der starr mit dem äußeren Teil des Schafts 20 verbunden sein kann. Dadurch kann der Platziergriff 32 benutzt werden, um das medizinische Instrument 1 in den menschlichen Körper einzuführen und am Operationsort zu platzieren.

Am Operationsgriffabschnitt 35 am proximalen Ende des Bedienabschnitts sind ein Okular 36 und ein Operationsgriff 37 angeordnet. Der Operateur (d.h. der Chirurg) ist nun in der Lage, seinen Daumen durch den Operationsgriff 37 zu führen und die übrigen Finger einer Hand durch den Platziergriff 32, um durch Öffnen und Schließen der Hand eine Vorwärts- und Rückwärtsbewegung eines Innenschafts relativ zu dem hohlen Außenschaft des Schaftes 20 auszuführen, sodass der Operateur mit nur einer Hand operieren kann.

Mithilfe des Operationsgriffes 37 können Teile des medizinischen Instruments 1 zusätzlich gedreht werden.

Folglich ist das medizinische Instrument 1 nach dieser Ausführungsform also ein Resektoskop mit einem zusätzlichen Aufsatz 100 zum mechanischen Abtragen von Gewebe. Jedoch kann der Aufsatz 100 auch an anderen, ähnlichen medizinischen Instrumenten angeordnet werden, insbesondere wenn diese den im Folgenden beschriebenen beweglichen Innenschaft 21 aufweisen.

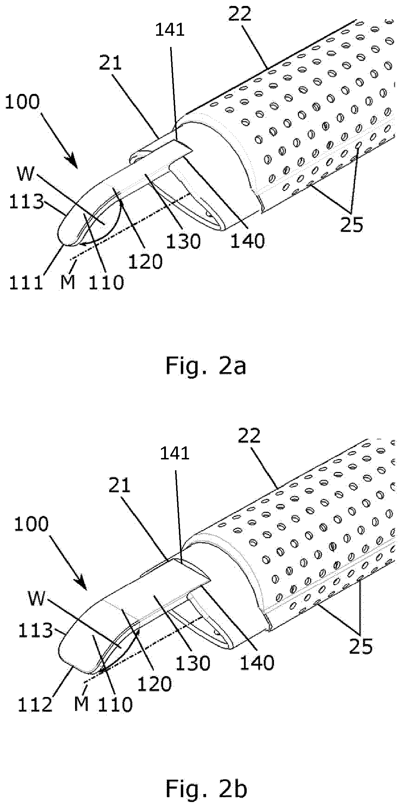

Fig. 2a zeigt eine detaillierte Ansicht des distalen Endes 10 einer bevorzugten Ausführungsform der vorliegenden Erfindung. Dabei ist ein Aufsatz 100 zum Abtragen von Gewebe am distalen Ende eines Innenschafts 21 angeordnet, der längsverschieblich in einem hohlen Außenschaft 22 geführt ist. Der zuvor erwähnte Schaft 20 umfasst also einen Außenschaft 22 und einen Innenschaft 21, der beweglich, insbesondere längsverschieblich, in dem Außenschaft 22 geführt ist. Der Außenschaft 22 weist an seinem vorderen Ende eine Vielzahl von Mündungslöchern 25 auf, die auf der Außenwand des Außenschafts 22 rund um diesen herum angeordnet sind. Durch die Mündungslöcher 25 wird eine Spülflüssigkeit rückgeführt.

Der Innenschaft 21 weist an seinem distalen Ende eine rechteckförmige Aussparung 141 auf, in der über einen Befestigungsabschnitt 140 ein korrespondierend ausgebildetes Ende eines Aufsatzes 100 zum Abtragen von Gewebe eingepasst und insbesondere eingeschweißt ist. Im Anschluss hieran erstreckt sich ein geradliniger Abschnitt 130 parallel zur Mittellinie M des Innenschaftes 21 hin zum distalen Ende. Dem geradlinigen Abschnitt 130 schließt sich ein abgewinkelter oder gekrümmter Abschnitt 110 an, der sich geneigt zum geradlinigen Abschnitt 130 erstreckt und dessen distales Ende eine Abtragekante 111 zum Abtragen von Gewebe ausbildet.

Bei der Ausführungsform nach der Fig. 2a ist die Abtragekante 111 seitlich symmetrisch abgerundet ausgebildet. Durch diese Anordnung kann eine aufgebrachte Kraft in einem kleinen vorderen Bereich der Abtragekante 111 konzentriert werden. Insbesondere kann die Abtragekante 111 gleichmäßig und symmetrisch gekrümmt ausgebildet sein. Diese Ausführungsform eignet sich also als Vorrichtung zum präzisen Abtragen von Gewebe. Insbesondere können gezielt Gewebefragmente entfernt werden. Der gesamte Aufsatz 100 ist dementsprechend (relativ) lang ausgebildet, um die Abtragekante 111 auch weit nach vorne zu führen. Hierzu liegt die axiale Länge des Aufsatzes 100 zum Abtragen von Gewebe im Bereich zwischen 1 mm und 16 mm, bevorzugter im Bereich zwischen 3 mm und 13 mm und noch bevorzugter im Bereich zwischen 5 mm und 11 mm.

Zusätzlich kann eine seitliche Abtragekante 113 auf einer der beiden Seiten des abgewinkelten Abschnitts 110 vorgesehen sein oder, auch zwei entsprechende Abtragekanten 113 auf beiden Seiten des abgewinkelten Abschnitts 110.

Fig. 2b zeigt eine detaillierte Ansicht des distalen Endes 10 der Ausführungsform nach der Fig. 1, bei der die Form des abgewinkelten Abschnitts 110 abweicht. Nach der Fig. 2b weist der abgewinkelte Abschnitt 110 eine Abtragekante 112 auf, die über den größten Teil geradlinig ausgebildet ist und am distalen Ende seitlich Abrundungen aufweist. Am distalen Ende des abgewinkelten Abschnitts 110 ist somit eine geradlinige Abtragekante 112 vorgesehen. Mit dieser Ausführungsform kann der Operateur eine Kraft auf einer breiteren Fläche aufbringen. Daher eignet sich diese Ausführungsform auch zum weiträumigen Abtragen von Gewebe. Dabei kann der Aufsatz in dieser Ausführungsform insbesondere relativ kurz ausgebildet sein, um diesen kontrollierbar zu machen.

Wie im obigen Ausführungsbeispiel kann bzw. können an dem abgewinkelten Abschnitt 110 zumindest eine entsprechende seitliche Abtragekante 113 vorgesehen sein, mit der ebenfalls Gewebe abgetrennt werden kann. Bei den vorgenannten Ausführungsformen kann der Übergangsbereich zwischen dem geradlinigen Abschnitt 130 und dem abgewinkelten Abschnitt 110 insbesondere bogenförmig gekrümmt ausgebildet sein.

Wie man den Figuren 2a und 2b entnehmen kann, schließt der gekrümmte oder abgewinkelte Abschnitt 110 mit dem gradlinigen Abschnitt 130 einen Winkel W im Bereich zwischen 115° und 155°, bevorzugter zwischen 120° und 150° und noch bevorzugter zwischen 130° und 140° ein. Das distale Ende des Innenschaftes 21 kann mit einer Senkrechten auf die Mittellinie M einen entsprechenden Winkel im Bereich zwischen 25° und 65°, bevorzugter zwischen 30° und 60° und noch bevorzugter zwischen 40° und 50° einschließen. Die Abtragekante 111, 112 erstreckt sich im Wesentlichen senkrecht zu einer Unterseite des distalen Endes des abgewinkelten oder bogenförmig gekrümmten Abschnitts 110, wodurch ein Ausschälen von Gewebe durch axiales Verstellen des medizinischen Instruments 1 ermöglicht ist, weil beim axialen Verstellen die Abtragekante 111, 112 unter einem Winkel von etwa 45° über das abzutragende Gewebe verschoben wird. Wie man den Figuren 2a und 2b entnehmen kann, erstreckt sich der Aufsatz 100 zum Abtragen von Gewebe bis maximal etwa 50% und bevorzugter bis maximal etwa 40% des Innendurchmessers des hohlen Außenschafts 22 radial einwärts, sodass durch Drehen des Außenschaftes 22 um die Mittellinie jeder beliebige Ort in axialer Verlängerung des Außenschaftes 22 erreicht werden kann. Dabei ist der Aufsatz 100 stets innerhalb eines von den Innenseiten des Außenschaftes 22 ausgebildeten Innenprofils angeordnet, was grundsätzlich ausreichend sein kann, um das distale Ende ohne Beschädigung von Gewebe in einen menschlichen Körper einzuführen, insbesondere dann, wenn der Aufsatz 100 nicht zu weit über das distale Ende des Innenschaftes 21 hinausragt.

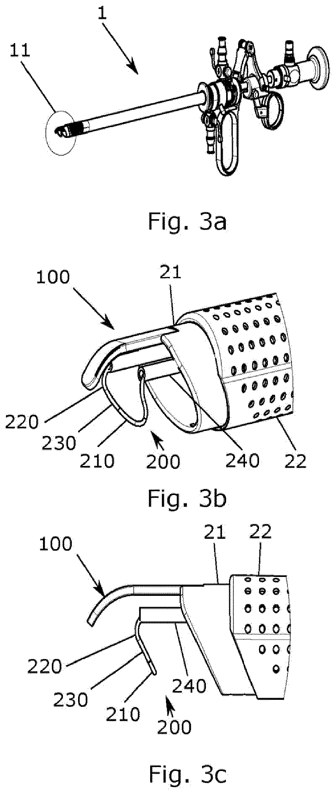

Fig. 3a zeigt ein medizinisches Instrument 1 nach einer weiteren Ausführungsform der vorliegenden Erfindung, bei der am distalen Ende 11 zusätzlich ein HF-Koagulationsaufsatz vorgesehen ist, dessen Einzelheiten in den stark vergrößerten Darstellungen nach den Figuren 3b und 3c gezeigt sind.

Wie in der Fig. 3b gezeigt, ist am distalen Ende des Innenschafts 21 proximal zum Aufsatz 100 zum Abtragen von Gewebe weiterhin ein HF-Koagulationsaufsatz 200 vorgesehen. Der HF-Koagulationsaufsatz 200 ist dabei von einer schleifenförmig ausgebildeten Schlinge ausgebildet, die mit einer Hochfrequenzspannung beaufschlagbar ist und einen unteren Schlingenabschnitt 210 und einen sich geneigt zur Instrumentenmittellinie erstreckenden mittleren Abschnitt 230 aufweist, der über einen oberen Verbindungsabschnitt 220 mit einer Stromzuleitung 240 verbunden ist. Der HF-Koagulationsaufsatz 200 ist über die Stromzuleitung mit einer Hochfrequenzspannung beaufschlagbar. Die Stromzuleitung 240 ist elektrisch gegen den Innenschaft 21 und den hohlen Außenschaft 22 isoliert, was auch für den HF-Koagulationsaufsatz 200 gilt.

Sowohl der obere Verbindungsabschnitt 220 als auch der geneigte, mittlere Verbindungsabschnitt 230 dienen dabei als Verbindungssegmente zum Platzieren des bogenförmig gekrümmten Schlingenabschnitts 210. Mit dem bogenförmig gekrümmten Schlingenabschnitt 210, ggf. zusätzlich mit dem geneigten, mittleren Verbindungsabschnitt 230 wird das eigentliche Koagulieren von Gewebe durchgeführt, indem die Schlinge mit einer Hochfrequenzspannung beaufschlagt wird.

Fig. 3c zeigt das distale Ende 11 nach der Fig. 3b nochmals in einer Seitenansicht, wobei die axiale und radiale Position des Aufsatzes 100 zum Abtragen von Gewebe in Relation zu dem HF-Koagulationsaufsatz 200 und dem Innenschaft 21 dargestellt sind. Der Aufsatz 100 zum Abtragen von Gewebe bildet das äußerste distale Ende des medizinischen Instruments 1. Proximal zum Aufsatz 100 zum Abtragen von Gewebe versetzt ist der HF-Koagulationsaufsatz 200 angeordnet, der zum Koagulieren von Gewebe weniger weit axial von dem Innenschaft 21 abragt als der Aufsatz 100 zum Abtragen von Gewebe. Somit schützt der Aufsatz 100 zum Abtragen von Gewebe in gewisser Weise den HF-Koagulationsaufsatz 200 vor mechanischen Beschädigungen, etwas beim axialen Verstellen des medizinischen Instruments 1 im Körpergewebe.

Auch in radialer Richtung sind der Aufsatz 100 zum Abtragen von Gewebe und der HF-Koagulationsaufsatz 200 beabstandet zueinander angeordnet, um eine elektrische Isolation zuverlässig zu bewerkstelligen. Sowohl der HF-Koagulationsaufsatz 200 als auch der Aufsatz 100 zum Abtragen von Gewebe sind nach unten, hin zur Instrumentenmittellinie des medizinischen Instruments zeigend, geneigt. Während der HF-Koagulationsaufsatz 200 mit dem geneigten, mittleren Abschnitt 230 unter einem spitzen Winkel zur Instrumentenmittellinie und hin zum distalen Endes des Innenschaftes 21 zeigend geneigt ist, erstreckt sich der Aufsatz 100 zum Abtragen von Gewebe schräg nach vorne, hin zum distalen Ende des medizinischen Instruments. Der Aufsatz 100 zum Abtragen von Gewebe erstreckt sich zweckmäßig nicht über die Instrumentenmittellinie hinaus, wohingegen der HF-Koagulationsaufsatz 200 zweckmäßig über ebendiese hinausragt, wie in der Fig. 3c gezeigt. Auch der HF-Koagulationsaufsatz 200 ist jedoch, in Vorderansicht betrachtet, innerhalb des Profils des Innenschaftes 21 angeordnet.

Selbstverständlich kann gemäß einer bevorzugten Ausführungsform nur der Aufsatz 100 zum mechanischen Abtragen von Gewebe vorgesehen sein und der HF-Koagulationsaufsatz 200 bei Bedarf zusätzlich an den Operationsort vorgeschoben werden, zu welchem Zweck das medizinische Instrument über einen weiteren Instrumentenport verfügt. Dabei kann der HF-Koagulationsaufsatz 200 starr und axial nicht verschieblich in dem Innenschaft 21 angeordnet sein. Gemäß einer bevorzugten Ausführungsform kann der HF-Koagulationsaufsatz 200 bei Bedarf, nämlich wenn mit einem optischen Einsatz, der in den Innenschaft 21 eingeführt ist, eine Beschädigung von Geweben und/oder eine Blutung festgestellt wird, unabhängig vom Innenschafts 21 axial verstellt und/oder gedreht werden, um exakt an dem Ort der Beschädigung / Blutung platziert zu werden. Hierzu kann der HF-Koagulationsaufsatz 200 als gesonderter Einsatz in den Innenschaft 21 eingeführt werden, beispielsweise gemeinsam mit dem optischen Einsatz.

Damit die axiale Verstellungslänge des HF-Koagulationsaufsatzes 200 minimiert ist, erstreckt sich der geneigte, mittlere Verbindungsabschnitt 230 bevorzugt parallel zum distalen Ende des Innenschaftes 21, wie in der Fig. 3c gezeigt.

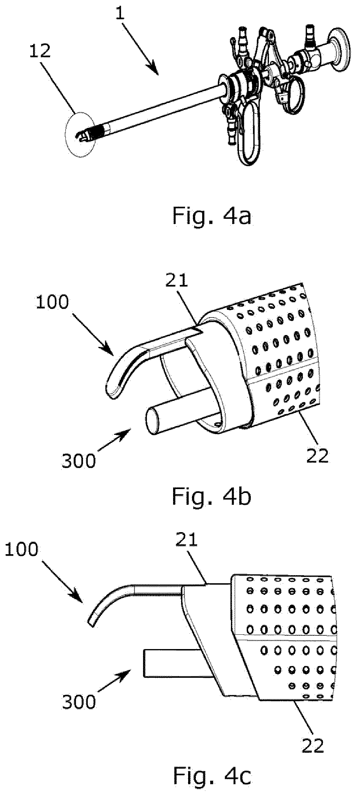

Die Figuren 4a bis 4c zeigen ein medizinisches Instrument 1 nach einer weiteren Ausführungsform der vorliegenden Erfindung, das zusätzlich zu dem Aufsatz 100 zum Abtragen von Gewebe einen Laser-Koagulationsaufsatz 300 aufweist.

Wie in der Fig. 4b gezeigt, ist am distalen Ende des Innenschaftes 21 proximal zum Aufsatz 100 zum Abtragen von Gewebe weiterhin ein Laser-Koagulationsaufsatz 300 vorgesehen. Der Laser-Koagulationsaufsatz 300 ragt dabei ebenfalls aus dem Innenschaft 21 heraus. In Bezug zur Mittellinie des hohlen Außenschafts 22 ist der Laser-Koagulationsaufsatz 300 diametral gegenüberliegend zum Aufsatz 100 zum mechanischen Abtragen von Gewebe angeordnet. Somit schützt der Aufsatz 100 zum Abtragen von Gewebe in gewisser Weise den Laser-Koagulationsaufsatz 300 vor mechanischen Beschädigungen, etwa beim axialen Verstellen des medizinischen Instruments 1 im Körpergewebe. Der Laser-Koagulationsaufsatz 300 kann insbesondere durch das distale Ende eines Lichtleiters ausgebildet sein, der zum distalen Ende des medizinischen Instruments 1 gerichtet ist, um Gewebe zu koagulieren. Hierzu kann der Laser-Koagulationsaufsatz 300 grundsätzlich auch innerhalb des Innenschaftes 21 an dessen distalem Ende angeordnet sein.

Selbstverständlich kann gemäß einer bevorzugten Ausführungsform nur der Aufsatz 100 zum mechanischen Abtragen von Gewebe vorgesehen sein und der Laser-Koagulationsaufsatz 300 bei Bedarf zusätzlich an den Operationsort vorgeschoben werden, zu welchem Zweck das medizinische Instrument über einen weiteren Instrumentenport verfügt. Dabei kann der Laser-Koagulationsaufsatz 300 starr und axial nicht verschieblich in dem Innenschaft 21 angeordnet sein. Gemäß einer bevorzugten Ausführungsform kann der Laser-Koagulationsaufsatz 300 bei Bedarf, nämlich wenn mit einem optischen Einsatz, der in den Innenschaft 21 eingeführt ist, eine Beschädigung von Geweben und/oder eine Blutung festgestellt wird, unabhängig vom Innenschafts 21 axial verstellt und/oder gedreht werden, um exakt an dem Ort der Beschädigung / Blutung platziert zu werden. Hierzu kann der Laser-Koagulationsaufsatz 300 als gesonderter Einsatz in den Innenschaft 21 eingeführt werden, beispielsweise gemeinsam mit dem optischen Einsatz.

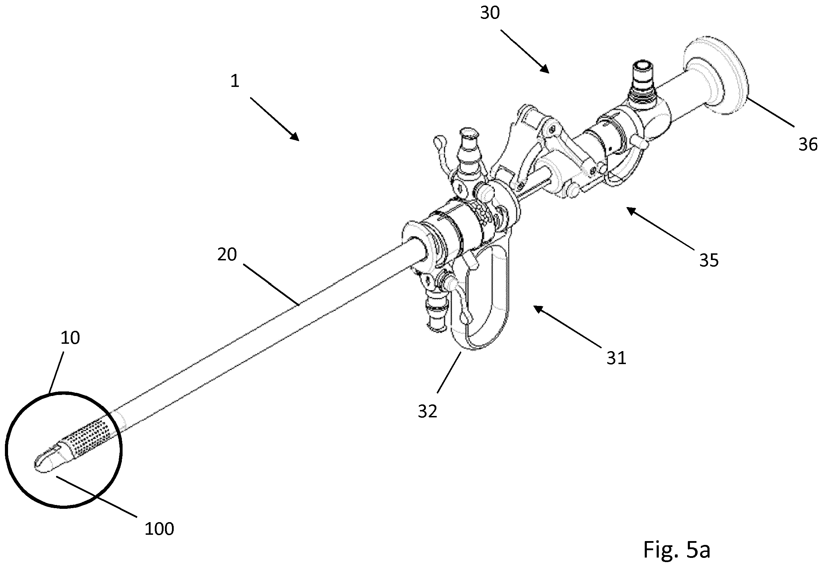

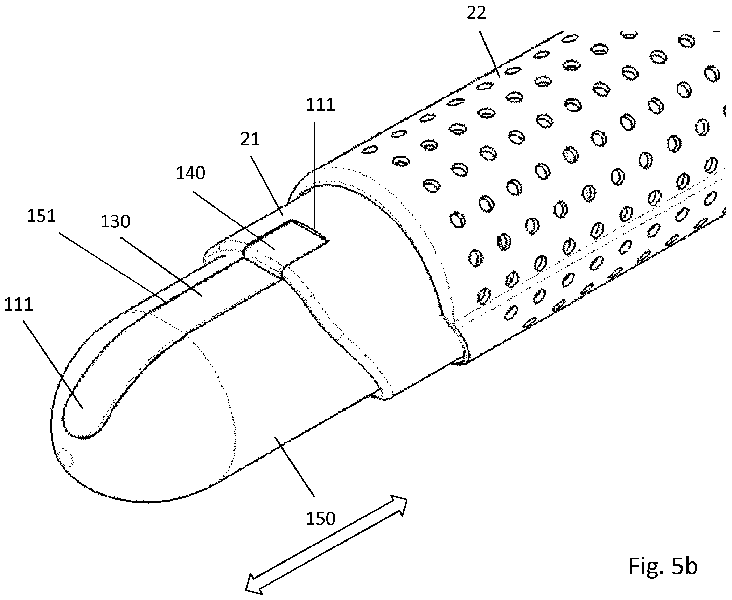

Die Figuren 5a und 5b zeigen ein medizinisches Instrument 1 gemäß einer weiteren Ausführungsform, bei der der Aufsatz 100 zum Abtragen von Gewebe zusätzlich abgedeckt werden kann, um eine unerwünschte Beschädigung von Gewebe zu verhindern, etwa beim Einführen des Instrumentenaufsatzes in menschliches Gewebe, beispielsweise in eine Harnröhre für Eingriffe an Prostatagewebe.

Wie in der Fig. 5b gezeigt, ist am distalen Ende des Innenschaftes 21 ein Aufnahmeelement bzw. Verschlussstück 150 vorgesehen, das relativ zu dem Innenschaft 21 axial verstellt werden kann, wie durch den Doppelpfeil angedeutet. Das distale Ende des Aufnahmeelements 150 ist ballig ausgebildet. Darin ist eine Aussparung 151 ausgebildet, die korrespondierend zur Form des Aufsatzes 100 zum Abtragen von Gewebe ausgebildet ist. In der ausgefahrenen Stellung gemäß der Fig. 5a ist der Aufsatz 100 zum Abtragen von Gewebe jedenfalls soweit in der Aussparung 151 aufgenommen, dass dessen Abtragekanten abgedeckt sind, insbesondere am Abschnitt 111, wie in der Fig. 5b dargestellt, wodurch eine unerwünschte Beschädigung von Gewebe verhindert werden kann, etwa beim Einführen des Instrumentenaufsatzes in menschliches Gewebe. Ein sanftes Einführen des Instrumentenaufsatzes in menschliches Gewebe wird dabei durch die ballige Form des distalen Endes des Aufnahmeelements 150 unterstützt.

Das Aufnahmeelement 150 kann axial soweit in den Innenschaft 21 zurückgefahren werden, dass der Aufsatz 100 zum Abtragen von Gewebe soweit freigelegt ist, um Gewebe abzutragen, etwa um Gewebe an der menschlichen Prostata durch axiales Verstellen des Innenschaftes 21 oder des gesamten Instrumentenaufsatzes mittels der Abtragekante 111 abzuschälen. Dabei kann das Aufnahmeelement 150 auch so ausgebildet sein, dass ein HF-Koagulationsaufsatz oder ein Laser-Koagulationsaufsatz in der zurückgefahrenen Stellung freigegeben sind, um Gewebe bei Bedarf auch koagulieren zu können.

Das Aufhahmeelement 150 kann insbesondere als Obturator ausgebildet sein, der das distale Ende des Instruments 1 beim Einführen in menschliches Gewebe vollständig verschließt und dabei den Aufsatz 100 zum Abtragen von Gewebe in ausreichendem Maße abdeckt oder aufnimmt, der jedoch anschließend auch vollständig aus dem hohlen Innenschafts 21 abgezogen werden kann, sodass dann beispielsweise ein optischer Einsatz, ein Koagulationseinsatz, wie vorstehend beschrieben, oder andere Instrumente in den hohlen Innenschaft 21 eingeführt werden können.

Für eine präzise Positionierung des Instrumentenaufsatzes ist der Innenschaft 21 bevorzugt relativ zu dem Außenschaft 22 geradgeführt, d.h. beim axialen Verstellen des Innenschaftes 21 relativ zu dem Außenschaft 22 wird der Innenschaft 21 nicht unkontrolliert verdreht. Dies kann grundsätzlich durch den formschlüssigen Eingriff von Führungsstrukturen am Innenschaft 21 mit korrespondierend ausgebildeten Führungsstrukturen am Außenschaft 22 erreicht werden, etwa durch den Eingriff von sich axial erstreckenden Führungsschienen oder Führungsleisten. Bevorzugt wird eine solche Geradführung jedoch automatisch durch das Profil von Innen- und Außenschaft 21, 22 selbst erzielt, wie in den Figuren 6a bis 7b gezeigt.

Gemäß der Fig. 6b weist der Innenschaft 21 ein elliptisches bzw. ovales Profil auf, das durch eine große Halbachse a und eine kleine Halbachse b festgelegt ist. Das Innenprofil des Außenschaftes (nicht gezeigt) ist korrespondierend hierzu ausgebildet. Beim axialen Verstellen des Innenschaftes 21 relativ zu dem Außenschaft wird so ein Verdrehen des Innenschaftes zuverlässig verhindert.

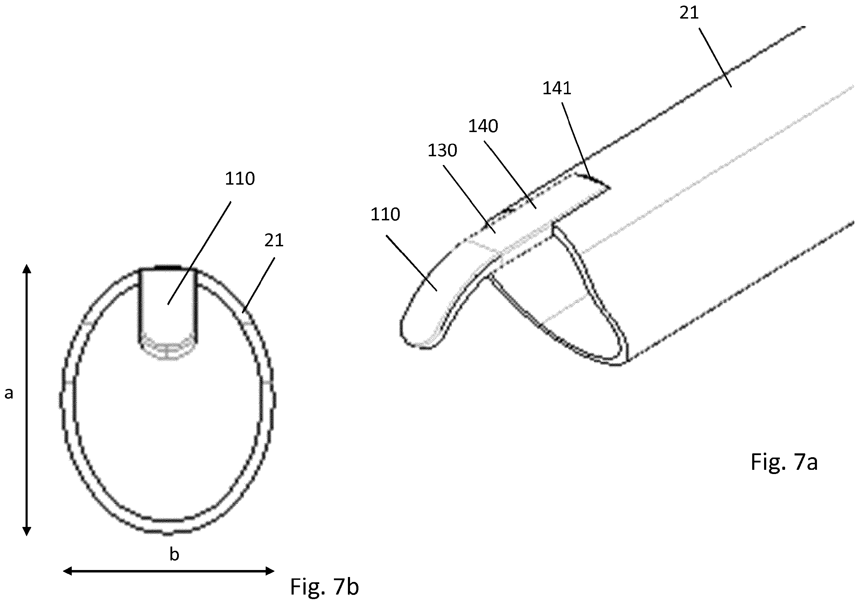

Gemäß dem Ausführungsbeispiel nach den Figuren 7a und 7b sind die Orientierungen der großen Halbachse a und der kleinen Halbachse b bei ansonsten gleicher Position und Orientierung des Aufsatzes 100 zum Abtragen von Gewebe gegenüber dem Ausführungsbeispiel nach den Figuren 6a und 6b vertauscht.

Im Folgenden wird die Handhabung des vorstehend beschriebenen medizinischen Instruments 1 bei einem operativen Einsatz beschrieben.

Zunächst wird ein medizinisches Instrument 1 bereitgestellt, wie vorstehend beschrieben, mit einem Aufsatz 100 zum mechanischen Abtragen von Gewebe, der unmittelbar am distalen Ende 10 des Innenschaftes 21 angeordnet ist, wobei der Aufsatz 100 innerhalb eines von den Innenseiten des Außenschaftes 22 ausgebildeten Innenprofils angeordnet ist und zum Abtragen von Gewebe das distale Ende des Innenschaftes 21 axial über das distale Ende des hohlen Außenschafts 22 hinausragt.

Anschließend wird der Schaft 20 in das menschliche Gewebe eingeführt, insbesondere in eine Harnröhre, um den Aufsatz 100 zum Abtragen von Gewebe in der Nähe der Prostata platzieren. Beim Einführen ist dabei der Aufsatz 100 zum Abtragen von Gewebe durch ein Aufnahmeelement 150, insbesondere einen Obturator, in ausreichendem Maße abgedeckt oder darin aufgenommen. Anschließend wird das Aufnahmeelement 150 bzw. der Obturator axial soweit zurückgefahren, dass der Aufsatz 100 zum Abtragen von Gewebe in ausreichendem Maße freiliegt. Durch axiales Verstellen des Schaftes 20, bevorzugt des Innenschaftes 21 gemeinsam mit dem daran vorgesehenen Aufsatz 100 zum Abtragen von Gewebe relativ zu dem hohlen Außenschaft 22, wird die Abtragekante des Aufsatzes 100 relativ zu dem Gewebe bewegt, wodurch insgesamt eine abschälende oder ausschälende Bewegung zum Abschälen oder Ausschälen von Gewebe, insbesondere von Prostatagewebe, ausgeführt wird.

Durch das Zurückfahren oder Herausfahren des Aufhahmeelements 150 bzw. des Obturators kann dabei ein HF- oder Laser-Koagulationsaufsatz, wie vorstehend beschrieben, in ausreichendem Maße freigelegt werden, um eine Koagulation von Gewebe durchführen zu können. Zur visuellen Kontrolle und Steuerung kann durch den hohlen Innenschaft 21 insbesondere eine geeignete Optik an den Operationsort geführt werden.

Nach dem Abtragen von Gewebe wird in umgekehrter Reihenfolge der Schaft 20 wieder aus dem menschlichen Gewebe abgezogen. Dabei ist der Aufsatz 100 zum Abtragen von Gewebe zweckmäßig erneut durch ein Aufnahmeelement 150, insbesondere einen Obturator, in ausreichendem Maße abgedeckt oder darin aufgenommen, um eine unerwünschte Beschädigung von Gewebe zu verhindern.

### Bezugszeichenliste

- 1: Medizinisches Instrument
- 10: distales Ende
- 11: distales Ende mit HF-Koagulationsaufsatz
- 12: distales Ende mit Laser-Koagulationsaufsatz

- 20: Schaft
- 21: Innenschaft
- 22: Außenschaft
- 23: Aufnahmeeinkerbung
- 25: Mündungslöcher

- 30: Bedienabschnitt
- 31: Platziergriffabschnitt
- 32: Platziergriff
- 35: Operationsgriffabschnitt
- 36: Okular
- 37: Operationsgriff

- 100: Aufsatz zum Abtragen von Gewebe
- 110: abgewinkelter Abschnitt / bogenförmig gekrümmter Abschnitt
- 111: symmetrisch gekrümmte Abtragekante
- 112: (im Wesentlichen) nicht gekrümmte Abtragekante
- 113: seitliche Abtragekanten
- 120: Abwinkelungstrenn- bzw. Knicklinie
- 130: sich geradlinig erstreckender Abschnitt
- 140: Befestigungsabschnitt
- 141: Aussparung

- 150: Obturator / Aufnahmeelement
- 151: Aussparung / Aufnahme

- 200: HF-Koagulationsaufsatz
- 210: unteres Ende von HF-Koagulationsschlinge
- 220: oberer Verbindungsabschnitt
- 230: geneigter / mittlerer Abschnitt von HF-Koagulationsschlinge
- 240: Stromzuleitung

- 300: Laser-Koagulationsaufsatz

- M: Instrumentenmittellinie
- W: Winkel

## Patentansprüche

1. Medizinisches Instrument (1) zum Abtragen von Gewebe durch einen minimal-invasiven chirurgischen Eingriff, umfassend
einen hohlen Außenschaft (22),
einen Innenschaft (21), und
einen Bedienabschnitt (30, 36) an einem proximalen Ende des medizinischen Instruments (1), um den hohlen Außenschaft (22) zu platzieren,
wobei ein Aufsatz (100) zum mechanischen Abtragen von Gewebe, durch axiales Verstellen des Innenschaftes (21) oder des Aufsatzes (100), unmittelbar an einem distalen Ende (10) des Innenschafts (21) angeordnet ist,
wobei der Aufsatz (100) nicht über ein von Innenseiten des Außenschafts (22) ausgebildetes Innenprofil hinausragt und wobei zum Abtragen von Gewebe das distale Ende des Aufsatzes (100) axial über das distale Ende des hohlen Außenschafts (22) hinausragt, wobei der Innenschaft (21) als hohles Rohr ausgebildet ist,
wobei das medizinische Instrument (1) weiterhin ein längliches Aufnahmeelement (150) mit einer Aussparung oder Aufnahme zur vorübergehenden Aufnahme des Aufsatzes (100) zum mechanischen Abtragen von Gewebe umfasst, wobei das längliche Aufhahmeelement (150) relativ zu dem Innenschaft (21) axial zwischen einer ausgefahrenen Stellung (Fig. 5b), in der der Aufsatz (100) zum mechanischen Abtragen von Gewebe in der Aussparung oder Aufnahme vorübergehend aufgenommen ist, und einer in den Innenschaft (21) eingefahrenen Stellung axial verstellbar ist, in der der Aufsatz (100) zum mechanischen Abtragen von Gewebe über das distale Ende des hohlen Außenschafts (22) axial hinausragt und zum Abtragen von Gewebe zugänglich ist.

2. Medizinisches Instrument (1) nach Anspruch 1, wobei der Aufsatz (100) zum mechanischen Abtragen von Gewebe über einen sich geradlinig erstreckenden Abschnitt (130) mit dem distalen Ende des Innenschafts (21) verbunden ist und ein distales Ende des Aufsatzes (100) sich geneigt zu dem geradlinigen Abschnitt (130) erstreckt.

3. Medizinisches Instrument (1) nach Anspruch 2, wobei sich dem geradlinigen Abschnitt ein bogenförmig gekrümmter Abschnitt (110) oder ein abgewinkelter Abschnitt anschließt, an dessen distalem Ende eine Abtragekante (111, 112) vorgesehen ist.

4. Medizinisches Instrument (1) nach Anspruch 3, wobei der gekrümmte oder abgewinkelte Abschnitt (110) mit dem gradlinigen Abschnitt (130) einen Winkel (W) im Bereich zwischen 115° und 155°, bevorzugter zwischen 120° und 150° und noch bevorzugter zwischen 130° und 140° einschließt.

5. Medizinisches Instrument (1) nach Anspruch 3 oder 4, wobei sich die Abtragekante (111, 112) im Wesentlichen senkrecht zu einer Unterseite des distalen Endes des abgewinkelten oder bogenförmig gekrümmten Abschnitts (110) erstreckt.

6. Medizinisches Instrument (1) nach Anspruch 3 bis 5, wobei die Abtragekante (112) einen zentralen Abschnitt aufweist, der nicht gekrümmt ausgebildet ist.

7. Medizinisches Instrument (1) nach Anspruch 3 bis 5, wobei die Abtragekante (111) einen zentralen Abschnitt aufweist, der symmetrisch gekrümmt ausgebildet ist.

8. Medizinisches Instrument (1) nach einem der vorherigen Ansprüche, wobei die axiale Länge des Aufsatzes (100) zum Abtragen von Gewebe im Bereich zwischen 1 mm und 16 mm, bevorzugter im Bereich zwischen 3 mm und 13 mm und noch bevorzugter im Bereich zwischen 5 mm und 11 mm liegt.

9. Medizinisches Instrument (1) nach einem der vorherigen Ansprüche, wobei sich der Aufsatz zum Abtragen von Gewebe bis maximal 50% und bevorzugter bis 40% des Innendurchmessers des hohlen Außenschafts (22) radial einwärts erstreckt.

10. Medizinisches Instrument (1) nach einem der vorherigen Ansprüche, wobei der Aufsatz (100) zum mechanischen Abtragen von Gewebe an das distale Ende des Innenschafts (21) angeschweißt ist.

11. Medizinisches Instrument (1) nach einem der vorhergehenden Ansprüche, wobei
der Innenschaft (21) längsverschieblich in dem hohlen Außenschaft (22) geführt ist, und
der Bedienabschnitt (30, 36) weiterhin ausgelegt ist, um die Position des Innenschaftes (21) relativ zu dem hohlen Außenschaft (22) zu verstellen.

12. Medizinisches Instrument (1) nach Anspruch 11, wobei der Bedienabschnitt (30) so mit dem Innenschaft (21) gekoppelt ist, dass der Aufsatz (100) vollständig in den hohlen Außenschaft (22) zurückgefahren werden kann und zum Abtragen von Gewebe zumindest abschnittsweise axial über ein distales Ende des hohlen Außenschafts hinausragt.

13. Medizinisches Instrument (1) nach Anspruch 11 oder 12, wobei der Innenschaft (21) in dem Außenschaft (22) längsverschieblich und geradgeführt ist.

14. Medizinisches Instrument (1) nach Anspruch 13, wobei ein Außenprofil des Innenschaftes (21) nicht rotationssymmetrisch ausgebildet ist, insbesondere als ovales oder elliptisches Außenprofil ausgebildet ist, und wobei ein Innenprofil des Außenschaftes (22) korrespondierend zum Außenprofil des Innenschaftes (21) ausgebildet ist, um den Innenschaft (21) bei einer axialen Verstellung geradzuführen.

15. Medizinisches Instrument (1) nach einem der vorherigen Ansprüche, wobei am distalen Ende des Innenschaftes (21) des Weiteren eine mit einer Hochfrequenzspannung beaufschlagbare Schlinge (200) zum Koagulieren von Gewebe vorgesehen ist, die axial weniger weit von dem distalen Ende des Innenschafts (21) vorsteht als der Aufsatz (100) zum mechanischen Abtragen von Gewebe und die zu dem Innenschaft elektrisch isoliert ist.

16. Medizinisches Instrument (1) nach Anspruch 15, wobei die Schlinge (200) einen Verbindungsabschnitt (240) und eine bogenförmig gekrümmte Schleife (210) am distalen Ende des Verbindungsabschnitts (240) aufweist, wobei sich die bogenförmig gekrümmte Schleife (210) im Wesentlichen parallel zum distalen Ende des Innenschafts (21) erstreckt.

17. Medizinisches Instrument (1) nach Anspruch 15, wobei in einer Seitenansicht des distalen Endes des medizinischen Instruments betrachtet, die bogenförmige Schleife (210) und das distale Ende des Innenschaftes (21) sich geneigt und hin zum proximalen Ende des hohlen Innenschafts erstrecken.

18. Medizinisches Instrument (1) nach einem der Ansprüche 14 bis 17, wobei die mit der Hochfrequenzspannung beaufschlagbare Schlinge (200) relativ zu dem Innenschaft (21) axial verstellbar ist.

19. Medizinisches Instrument (1) nach einem der vorherigen Ansprüche, wobei am distalen Ende des Innenschaftes (21) des Weiteren ein Laser-Koagulationsaufsatz (300) zum Koagulieren von Gewebe vorgesehen ist, der axial von dem distalen Ende des Innenschafts (21) vorsteht.

20. Medizinisches Instrument (1) nach Anspruch 19, wobei der Laser-Koagulationsaufsatz (300) weniger weit von dem distalen Ende des Innenschafts (21) vorsteht als der Aufsatz (100) zum mechanischen Abtragen von Gewebe.

## Claims

1. Medical instrument (1) for tissue ablation by a minimally invasive surgical procedure, comprising
a hollow outer shaft (22),
an inner shaft (21), and
a control portion (30, 36) at a proximal end of the medical instrument (1), in order to position the hollow outer shaft (22),
wherein an attachment (100) for mechanical ablation of tissue, by axial adjustment of the inner shaft (21) or of the attachment (100), is arranged directly at a distal end (10) of the inner shaft (21),
wherein the attachment (100) does not protrude beyond an inner profile formed by inner faces of the outer shaft (22), and wherein the distal end of the attachment (100) protrudes axially beyond the distal end of the hollow outer shaft (22) in order to ablate tissue,
wherein the inner shaft (21) is configured as a hollow tube,
wherein the medical instrument (100) further comprises an elongate receiving element (150) with a cutout or seat for temporarily receiving the attachment (100) for mechanical ablation of tissue, wherein the elongate receiving element (150) is axially adjustable relative to the inner shaft (21) between a deployed position (Fig. 5b), in which the attachment (100) for mechanical ablation of tissue is temporarily received in the cutout or seat, and a retracted position in the inner shaft (21), in which the attachment (100) for mechanical ablation of tissue protrudes axially beyond the distal end of the hollow outer shaft (22) and is accessible for tissue ablation.

2. Medical instrument (1) according to Claim 1, wherein the attachment (100) for mechanical ablation of tissue is connected to the distal end of the inner shaft (21) via a rectilinearly extending portion (130), and a distal end of the attachment (100) extends at an inclination to the rectilinear portion (130).

3. Medical instrument (1) according to Claim 2, wherein the rectilinear portion is adjoined by an arcuately curved portion (110) or an angled portion at whose distal end an ablation edge (111, 112) is provided.

4. Medical instrument (1) according to Claim 3, wherein the curved or angled portion (110) encloses an angle (W) of between 115° and 155°, preferably of between 120° and 150°, more preferably of between 130° and 140°, with the rectilinear portion (130).

5. Medical instrument (1) according to Claim 3 or 4, wherein the ablation edge (111, 112) extends substantially perpendicularly with respect to an underside of the distal end of the angled or arcuately curved portion (110).

6. Medical instrument (1) according to Claims 3 to 5, wherein the ablation edge (112) has a central portion which is not curved.

7. Medical instrument (1) according to Claims 3 to 5, wherein the ablation edge (111) has a central portion which is curved symmetrically.

8. Medical instrument (1) according to one of the preceding claims, wherein the axial length of the attachment (100) for tissue ablation lies in the range of between 1 mm and 16 mm, preferably in the range of between 3 mm and 13 mm, more preferably in the range of between 5 mm and 11 mm.

9. Medical instrument (1) according to one of the preceding claims, wherein the attachment for tissue ablation extends radially inward by at most 50% and preferably 40% of the internal diameter of the hollow outer shaft (22).

10. Medical instrument (1) according to one of the preceding claims, wherein the attachment (100) for mechanical ablation of tissue is welded onto the distal end of the inner shaft (21).

11. Medical instrument (1) according to one of the preceding claims, wherein the inner shaft (21) is guided longitudinally displaceably in the hollow outer shaft (22), and the control portion (30, 36) is moreover configured for adjusting the position of the inner shaft (21) relative to the hollow outer shaft (22).

12. Medical instrument (1) according to Claim 11, wherein the control portion (30) is coupled to the inner shaft (21) such that the attachment (100) can be retracted fully into the hollow outer shaft (22) and, in order to permit tissue ablation, at least partially protrudes axially beyond a distal end of the hollow outer shaft.

13. Medical instrument (1) according to Claim 11 or 12, wherein the inner shaft (21) is guided longitudinally displaceably and rectilinearly in the outer shaft (22).

14. Medical instrument (1) according to Claim 13, wherein an outer profile of the inner shaft (21) is not rotationally symmetrical, being configured in particular as an oval or elliptical outer profile, and wherein an inner profile of the outer shaft (22) is configured corresponding to the outer profile of the inner shaft (21) in order to guide the inner shaft (21) rectilinearly during an axial adjustment.

15. Medical instrument (1) according to one of the preceding claims, wherein a coil (200) to which a high-frequency voltage can be applied for tissue coagulation is moreover provided at the distal end of the inner shaft (21), which coil (200) protrudes axially from the distal end of the inner shaft (21) to a lesser extent than the attachment (100) for mechanical ablation of tissue and is electrically insulated from the inner shaft.

16. Medical instrument (1) according to Claim 15, wherein the coil (200) has a connecting portion (240) and an arcuately curved loop (210) at the distal end of the connecting portion (240), wherein the arcuately curved loop (210) extends substantially parallel to the distal end of the inner shaft (21).

17. Medical instrument (1) according to Claim 15, wherein, seen in a side view of the distal end of the medical instrument, the arc-shaped loop (210) and the distal end of the inner shaft (21) extend at an inclination and toward the proximal end of the hollow inner shaft.

18. Medical instrument (1) according to one of Claims 14 to 17, wherein the coil (200) to which the high-frequency voltage can be applied is axially adjustable relative to the inner shaft (21).

19. Medical instrument (1) according to one of the preceding claims, wherein a laser coagulation attachment (300) for tissue coagulation is moreover provided at the distal end of the inner shaft (21) and protrudes axially from the distal end of the inner shaft (21).

20. Medical instrument (1) according to Claim 19, wherein the laser coagulation attachment (300) protrudes from the distal end of the inner shaft (21) less far than the attachment (100) for mechanical ablation of tissue.

## Revendications

1. Instrument médical (1) destiné à enlever du tissu par le biais d'une intervention chirurgicale minimalement invasive, ledit instrument comprenant
une tige extérieure creuse (22),
une tige intérieure (21), et
une portion de manipulation (30, 36) à une extrémité proximale de l'instrument médical (1) afin de placer la tige extérieure creuse (22),
un appendice (100) destiné à enlever mécaniquement du tissu par déplacement axialement de la tige intérieure (21) ou de l'appendice (100) étant disposé directement à une extrémité distale (10) de la tige intérieure (21),
l'appendice (100) ne faisant pas saillie d'un profil intérieur formé par des côtés intérieurs de la tige extérieure (22) et l'extrémité distale de l'appendice (100) faisant saillie axialement de l'extrémité distale de la tige extérieure creuse (22) pour enlever du tissu, la tige intérieure (21) étant conçue sous la forme d'un tube creux,
l'instrument médical (1) comprenant en outre un élément de réception allongé (150) pourvu d'un évidement ou d'un réceptacle destiné à recevoir temporairement l'appendice (100) destiné à enlever mécaniquement du tissu, l'élément de réception allongé (150) étant déplaçable axialement par rapport à la tige intérieure (21) axialement entre une position déployée (figure 5b), dans laquelle l'appendice (100) destiné à enlever mécaniquement du tissu est logé temporairement dans l'évidement ou le réceptacle, et une position, rétractée dans la tige intérieure (21), dans laquelle l'appendice (100) destiné à enlever mécaniquement du tissu fait saillie axialement de l'extrémité distale de la tige extérieure creuse (22) et est accessible pour enlever du tissu.

2. Instrument médical (1) selon la revendication 1, l'appendice (100) destiné à enlever mécaniquement du tissu étant relié à l'extrémité distale de la tige intérieure (21) par le biais d'une portion (130) s'étendant en ligne droite et une extrémité distale de l'appendice (100) s'étendant de manière inclinée par rapport à la portion rectiligne (130).

3. Instrument médical (1) selon la revendication 2, la portion rectiligne étant raccordée à une portion (110) incurvée en arc ou une portion coudée à l'extrémité distale de laquelle est prévu une arête d'enlèvement (111, 112).

4. Instrument médical (1) selon la revendication 3, la portion incurvée ou coudée (110) formant avec la portion rectiligne (130) un angle (W) compris entre 115° et 155°, de préférence entre 120° et 150° et plus préférablement entre 130° et 140°.

5. Instrument médical (1) selon la revendication 3 ou 4, l'arête d'enlèvement (111, 112) s'étendant sensiblement perpendiculairement à un côté inférieur de l'extrémité distale de la portion coudée ou incurvée (110).

6. Instrument médical (1) selon la revendication 3 à 5, l'arête d'enlèvement (112) comportant une portion centrale qui n'est pas incurvée.

7. Instrument médical (1) selon la revendication 3 à 5, l'arête d'enlèvement (111) comportant une portion centrale qui est incurvée de manière symétrique.

8. Instrument médical (1) selon l'une des revendications précédentes, la longueur axiale de l'appendice (100) destiné à enlever du tissu étant comprise entre 1 mm et 16 mm, de préférence entre 3 mm et 13 mm et plus préférablement entre 5 mm et 11 mm.

9. Instrument médical (1) selon l'une des revendications précédentes, l'appendice destiné à enlever du tissu s'étendant radialement vers l'intérieur jusqu'à un maximum de 50 % et plus préférablement jusqu'à 40 % du diamètre intérieur de la tige extérieure creuse (22).

10. Instrument médical (1) selon l'une des revendications précédentes, l'appendice (100) destiné à enlever mécaniquement du tissu étant soudé à l'extrémité distale de la tige intérieure (21).

11. Instrument médical (1) selon l'une des revendications précédentes, la tige intérieure (21) étant guidée de manière à coulisser longitudinalement dans la tige extérieure creuse (22),
et
la portion de manipulation (30, 36) étant en outre conçue pour régler la position de la tige intérieure (21) par rapport à la tige extérieure creuse (22).

12. Instrument médical (1) selon la revendication 11, la portion de manipulation (30) étant accouplée à la tige intérieure (21) de telle manière que l'appendice (100) puisse être complètement rétracté dans la tige extérieure creuse (22) et fasse saillie axialement au moins par portions d'une extrémité distale de la tige extérieure creuse pour enlever du tissu.

13. Instrument médical (1) selon la revendication 11 ou 12, la tige intérieure (21) étant guidée de manière rectiligne et pouvant coulisser longitudinalement dans la tige extérieure (22).

14. Instrument médical (1) selon la revendication 13, un profil extérieur de la tige intérieure (21) n'étant pas conçu à symétrie de révolution, en particulier étant conçu comme un profil extérieur ovale ou elliptique, et un profil intérieur de la tige extérieure (22) étant conçu de manière à correspondre au profil extérieur de la tige intérieure (21) afin de guider la tige intérieure (21) de manière rectiligne lors d'un déplacement axial.

15. Instrument médical (1) selon l'une des revendications précédentes, un lacet (200), destiné à coaguler le tissu et pouvant être soumis à une tension à haute fréquence, étant en outre prévu à l'extrémité distale de la tige intérieure (21), lequel lacet fait moins saillie axialement de l'extrémité distale de la tige intérieure (21) que l'appendice (100) destiné à enlever mécaniquement du tissu et est isolé électriquement de la tige intérieure.

16. Instrument médical (1) selon la revendication 15, le lacet (200) comportant une portion de liaison (240) et une boucle (210) incurvée en arc à l'extrémité distale de la portion de liaison (240), la boucle (210) incurvée en arc s'étendant sensiblement parallèlement à l'extrémité distale de la tige intérieure (21).

17. Instrument médical (1) selon la revendication 15, dans une vue latérale de l'extrémité distale de l'instrument médical, la boucle (210) incurvée en arc et l'extrémité distale de la tige intérieure (21) s'étendant de manière inclinée et en direction l'extrémité proximale de la tige intérieure creuse.

18. Instrument médical (1) selon l'une des revendications 14 à 17, le lacet (200) qui peut être soumis à la tension à haute fréquence étant réglable axialement par rapport à la tige intérieure (21).

19. Instrument médical (1) selon l'une des revendications précédentes, un appendice de coagulation par laser (300) destiné à coaguler du tissu étant en outre prévu à l'extrémité distale de la tige intérieure (21), lequel appendice fait saillie axialement de l'extrémité distale de la tige intérieure (21).

20. Instrument médical (1) selon la revendication 19, l'appendice de coagulation par laser (300) faisant moins saillie de l'extrémité distale de la tige intérieure (21) que l'appendice (100) destiné à enlever mécaniquement du tissu.
